# EUROPEAN PATENT SPECIFICATION

(11) **EP 1 276 497 B1**
(45) Date of publication and mention of the grant of the patent: **23.11.2005**
(21) Application number: 01931582.9
(22) Date of filing: 10.04.2001
(51) Int. Cl.: A61K 38/19, A61P 7/00, C12N 5/06

(54) **MIXTURE OF DEFIBROTIDE AND G-CSF AND ITS USE FOR ACTIVATING HAEMATOPOIETIC PROGENITORS**
KOMBINATION VON DEFIBROTID UND G-CSF UND IHRE VERWENDUNG ZUR AKTIVIERUNG HÄMATOPOIETISCHER VORLÄUFERZELLEN
COMPOSITION DE DEFIBROTIDE ET DE G-CSF ET SON UTILISATION POUR L'ACTIVATION DES PROGENITEURS HEMATOPOIETIQUES

(30) Priority: 18.04.2000 EP 00830293
(43) Date of publication of application: 22.01.2003
(60) Divisional application: 05108424.2
(73) Proprietor: GENTIUM SPA, I-22089 Villa Guardia (IT)
(72) Inventor: FERRO, Laura, I-20100 Milano (IT); PORTA, Roberto, I-22012 Cernobbio (IT); IACOBELLI, Massimo, I-20152 Milano (IT); GIANNI, Alessandro, Massimo, I-20135 Milano (IT); STELLA, Carmelo, Carlo, I-20129 Milano (IT)
(74) Representative: Pistolesi, Roberto
(86) International application number: PCT/EP2001/004105
(87) International publication number: WO 2001/078761

(56) References cited:
- EP-A- 0 317 766
- EP-A- 0 416 678
- WO-A-99/26639
- US-A- 5 199 942
- US-A- 5 977 083
- GURSOY A: "PREPARATION, CHARACTERIZATION AND ANTI-INFLAMMATORY EFFECT OF DEFIBROTIDE LIPOSOMES" PHARMAZIE,DD,VEB VERLAG VOLK UND GESUNDHEIT. BERLIN, vol. 48, no. 7, 1 July 1993 (1993-07-01), pages 549-550, XP000372658 ISSN: 0031-7144
- CARLO-STELLA, C. (1) ET AL: "Defibrotide significantly enhances peripheral blood progenitor cell mobilization induced by recombinant human granulocyte colony - stimulating factor ( rhG - CSF." BLOOD, ( NOVEMBER 16, 2000 ) VOL. 96, NO. 11 PART 1, PP. 553A. PRINT. MEETING INFO.: 42ND ANNUAL MEETING OF THE AMERICAN SOCIETY OF HEMATOLOGY SAN FRANCISCO, CALIFORNIA, USA DECEMBER 01-05, 2000 AMERICAN SOCIETY OF HEMATOLOGY. , XP002176349

## Description

The present invention relates to a novel formulation capable of increasing the amount of stem cells and progenitor cells in circulation in the peripheral blood of a mammal; the formulation is characterised in that it contains defibrotide in combination with at least one haematopoietic factor having the capacity to mobilise haematopoietic progenitors, preferably G-CSF.

### FIELD OF THE INVENTION

The possibility of obtaining an increased amount of stem cells and haematopoietic progenitors in circulation in the peripheral blood of a mammal, and in particular in that of a human being, has for years been the subject of intensive research activity; the availability of stem cells and/or haematopoietic progenitors is in fact particularly important in sectors such as the autologous transplantation of circulating haematopoietic progenitors, the allotransplantation of circulating haematopoietic progenitors and in program for the gene therapy of circulating haematopoietic cells.

Although, after birth, stem cells and progenitor cells are located almost exclusively in the bone marrow, they nevertheless exhibit migratory properties; that is to say, under physiological conditions, they migrate through the cavities of the bone marrow and pass into circulation. That process, commonly known as "mobilisation", can be amplified in mammals by various treatments, such as, for example, the administration of cytokines and, in particular, the growth factor of granulocyte colonies (G-CSF); the reverse process, known as "homing", occurs, for example, in irradiated receivers after the transplantation of haematopoietic cells; the mechanisms on which mobilisation and homing are based are, however, still obscure (C.F. Craddock et al., "Antibodies to VLA4 Integrin Mobilize Long-Term Repopulating Cells and Augment Cytokine-Induced Mobilization in Primates and Mice", Blood, Vol. 90, n. 12, 1997, pp. 4779-4788; F. Prosper et al., "Mobilization and Homing of Peripheral Blood Progenitors is Related to Reversible Downregulation of α4β1 Integrin Expression and Function", J. Clin. Invest., Vol. 101, n. 11, 1998, pp. 2456-2467; M. Vermeulen et al., "Role of Adhesion Molecules in the Homing and Mobilization of Murine Hematopoietic Stem and Progenitor Cells", Blood, Vol. 92, n.3, 1998, pp. 894-900).

G-CSF (CAS registry number 143011-2-7/Merck Index, 1996, page 4558) is a haematopoietic growth factor which is indispensable in the proliferation and differentiation of the progenitor cells of granulocytes; it is a 18-22 kDa glycoprotein normally produced in response to specific stimulation by a variety of cells, including monocytes, fibroblasts and endothelial cells. The term defibrotide (CAS registry number 83712-60-1) normally identifies a polydeoxyribonucleotide obtained by extraction (US 3,770,720 and US 3,899,481) from animal and/or vegetable tissue; this polydeoxyribonucleotide is normally used in the form of a salt of an alkali metal, generally sodium. Defibrotide is used principally for its antithrombotic activity (US 3,829,567) although it may be used in different applications, such as, for example, the treatment of acute renal insufficiency (US 4,694,134) and the treatment of acute myocardial ischaemia (US 4,693,995). United States patents US 4,985,552 and US 5,223,609, finally, describe a process for the production of defibrotide which enables a product to be obtained which has constant and well defined physico-chemical characteristics and is also free from any undesired side-effects.

For the purposes of the present invention, the term defibrotide should therefore be understood as meaning any oligonucleotide and/or polynucleotide obtained by extraction from animal and/or vegetable tissue, in particular from the organs of mammals. Preferably, defibrotide is produced in accordance with the methods described in the patents listed above which should thus be regarded as an integral part of the present description; even more preferably, it is produced in accordance with the method described in United States patents US 4,985,552 and US 5,223,609.

### DETAILED DESCRIPTION OF THE INVENTION

It has now surprisingly been found that it is possible to obtain increased mobilisation of stem cells and haematopoietic progenitors by the administration of defibrotide in combination and/or in close temporal proximity with a haematopoietic factor having the capacity to mobilise haematopoietic progenitors.

As will be appreciated from the Examples, the administration of defibrotide in combination and/or in close temporal proximity with a haematopoietic factor having the capacity to mobilise haematopoietic progenitors permits the attainment of mobilisation levels much higher than those obtainable by the administration of the haematopoietic factor alone.

The subject of the present invention is therefore represented by a formulation containing as "active agents" defibrotide in combination with at least one haematopoietic factor having the capacity to mobilise haematopoietic progenitors, preferably G-CSF. In its preferred embodiment, this formulation is constituted by an injectable aqueous solution; alternatively, the formulation could be constituted by two different solutions, one containing defibrotide and the other containing the haematopoietic factor having the capacity to mobilise haematopoietic progenitors.

The formulation according to the present invention is therefore shaped as a combined preparation for simultaneous, separate or sequential use of the aforementioned active principles in order to increase the amount of stem cells and/or haematopoietic progenitors in circulation in the peripherial blood of a mammal.

A second subject of the present invention is represented by the use of defibrotide, in combination with at least one haematopoietic factor having the capacity to mobilise haematopoietic progenitors, for the preparation of formulations capable of increasing the amount of stem cells and/or haematopoietic progenitors in circulation in the peripheral blood of a mammal, preferably a human being.

Finally, the present invention makes possible a method of increasing the amount of stem cells and/or haematopoietic progenitors in circulation in the peripheral blood of a mammal, characterised in that defibrotide is administered to the mammal in combination or in temporal proximity with at least one haematopoietic factor having the capacity to mobilise haematopoietic progenitors. The haematopoietic factor used to conduct the experiments which led to the present invention is G-CSF; however, it is not to be excluded that similar results may be obtained with haematopoietic factors other than G-CSF but nevertheless having the capacity to mobilise haematopoietic progenitors, such as, for example, the growth factor of granulocyte and macrophage colonies (GM-CSF), "Flt3 ligand" (FL), "stem cell factor" (SCF), thrombopoietin (TPO), interleukin 8 (IL-8), and others which will be clear to persons skilled in the art.

The defibrotide used in combination with G-CSF in this first experimental stage was the defibrotide currently marketed by Crinos Spa under the mark Prociclide™ and produced in accordance with the process described in United States patents US 4,985,552 and US 5,223,609.

Defibrotide and haematopoietic factor having the capacity to mobilise haematopoietic progenitors can be administered to mammals (and in particular to human beings) in accordance with the methods and the posologies known in the art; generally, they are administered orally, intramuscularly, intraperitoneally, subcutaneously or intravenously, the last-mentioned route being the preferred one.

The two active ingredients can also be administered simultaneously or in succession. That is to say, in the first case, they are administered by means of a single formulation which contains both of the active ingredients and to which the usual excipients and/or coadjuvants known in the art have optionally been added; alternatively, the two active ingredients may be administered sequentially, namely, by means of two different formulations, one containing the haematopoietic factor having the capacity to mobilise haematopoietic progenitors, preferably G-CSF, and the other containing the defibrotide.

Generally, G-CSF will be administered subcutaneously, at a dosage of 5 to 24 µg/kg whereas DEF will be administered by continuous infusion at a dosage of 5 to 15 mg/kg/hr for 2-7 days.

As will be appreciated from the accompanying Examples, which are to be regarded purely as non-limiting illustrations of the invention, the combined administration of G-CSF and defibrotide to mice, as the most common experimental mammal model, and to monkeys, permits the attainment of levels of mobilisation much higher than those obtainable by the administration of G-CSF alone, with clear advantages for all those therapeutic sectors for which a high level of mobilisation is desirable.

### EXAMPLE 1

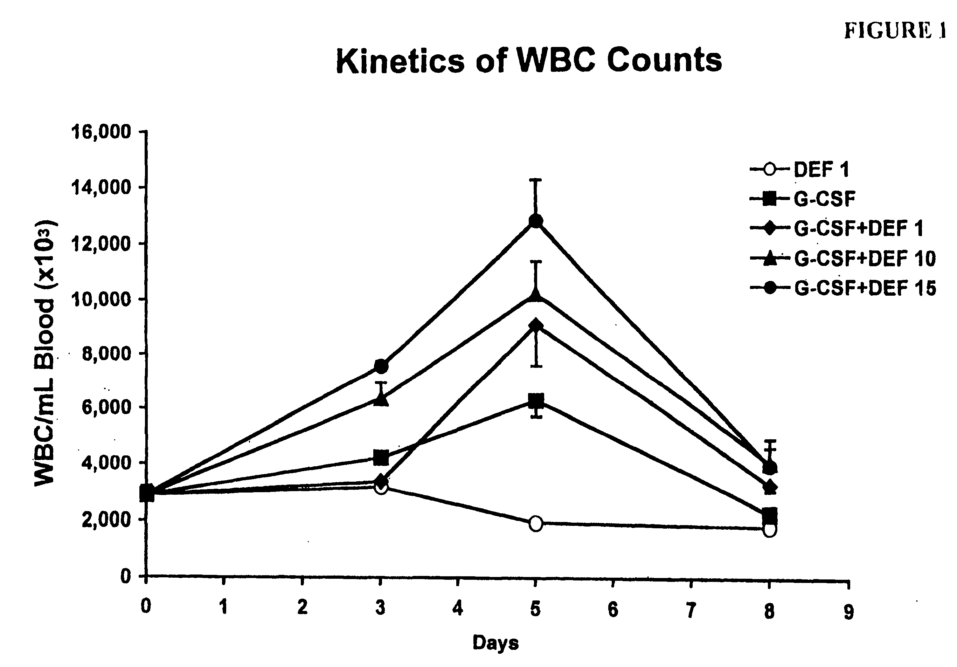

This experiment was carried out to evaluate the effect of the administration of G-CSF and/or defibrotide (DEF) on the amount of white blood cells (WBC) present in murine blood. BALB/c mice from 6 to 8 weeks old and having a body weight of from 20 to 25 g were subjected to intraperitoneal (IP) injections of G-CSF (5 µg/mouse/day), DEF (1 mg/mouse/day), or a combination of G-CSF (5 µg /mouse/day) and increasing doses of DEF (1, 10, 15 mg/mouse/day). A saline solution, buffered to 0.1%, of murine serum albumin (PBS/MSA) was administered by IP injection to the control mice which had not received G-CSF and/or DEF. The mice were treated for 5 days and sacrificed after 3 or 5 days of treatment, or 3 days after therapy had ceased. The results of this experiment are given in Figure 1.

The following symbols were used to represent each group of mice: G-CSF (■) (n=24), DEF 1 (O) (n=3), G-CSF+DEF 1 (◆) (n=13), G-CSF+DEF 10 (▲) (n=6), G-CSF+DEF 15 (●) (n=23). The mean white blood cell count in PBS/MSA in the control mice was 2.87 ± 0.2 x 10⁶/ml of blood; the data are expressed as mean ± standard error of the mean (SEM).

### EXAMPLE 2

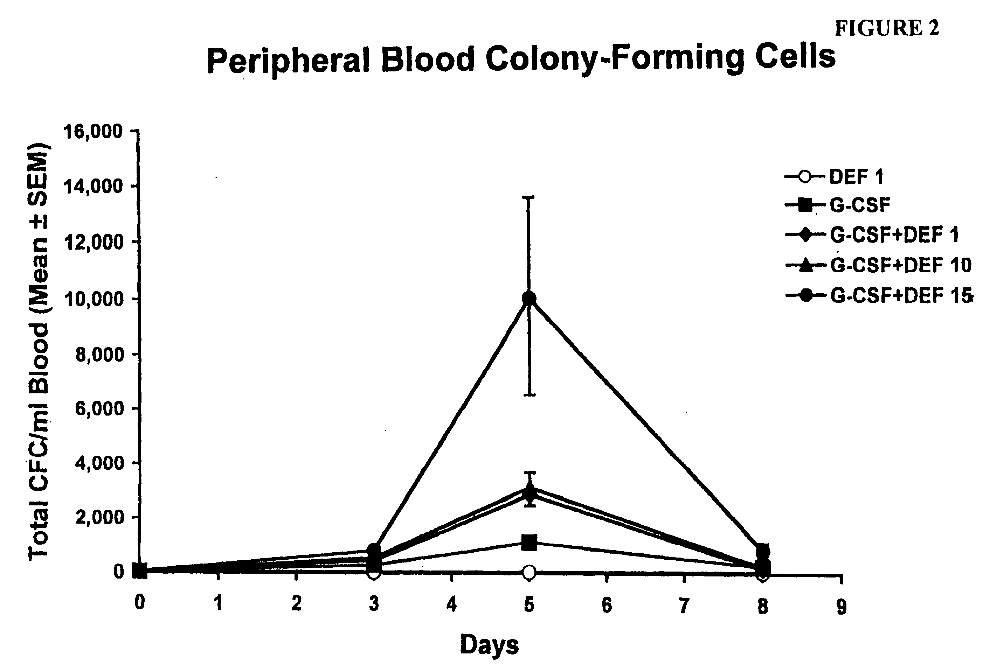

Mobilisation kinetics of the cells forming the total colonies (CFC) per millilitre of blood of the mice of Example 1. The mean CFC count in PBS/MSA in the control mice was 39 ± 12 per ml of blood; the data are given in Figure 2 and are expressed as mean ± SEM derived from duplicated cultures on samples from each animal at each point in time.

### EXAMPLE 3

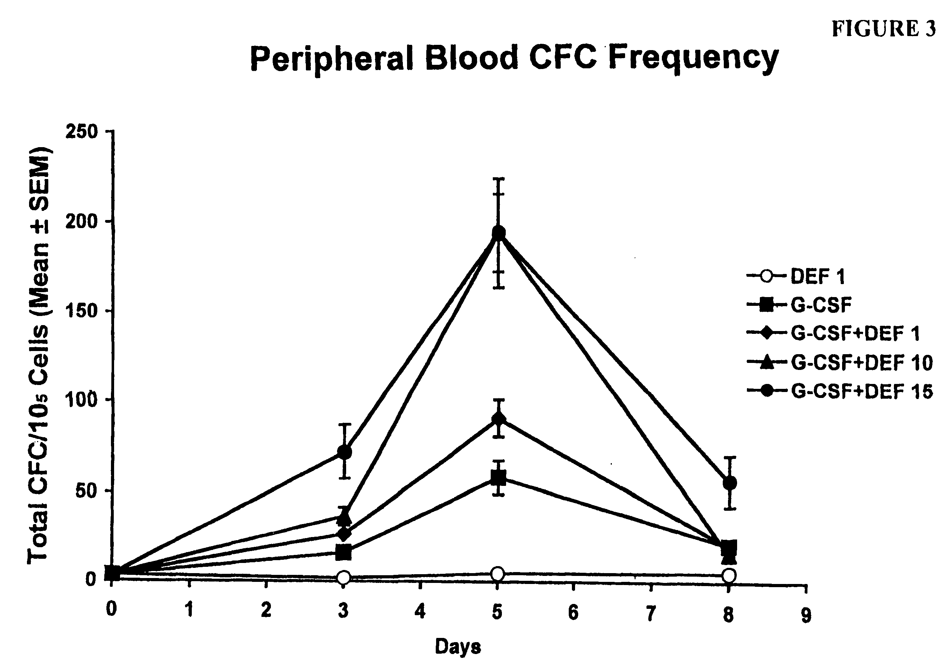

Changes in the frequency of the total CFCs (CFU-GM + BFU-E + CFU-Mix + HPP-CFC) per 10⁵ buffy-coat cells of peripheral blood in the mice mentioned in Example 1. The mean CFC count in PBS/MSA in the control mice was 3.5 ± 1; the data are given in Figure 3 and are expressed as mean ± SEM derived from duplicated cultures on samples from each animal at each point in time.

### EXAMPLE 4

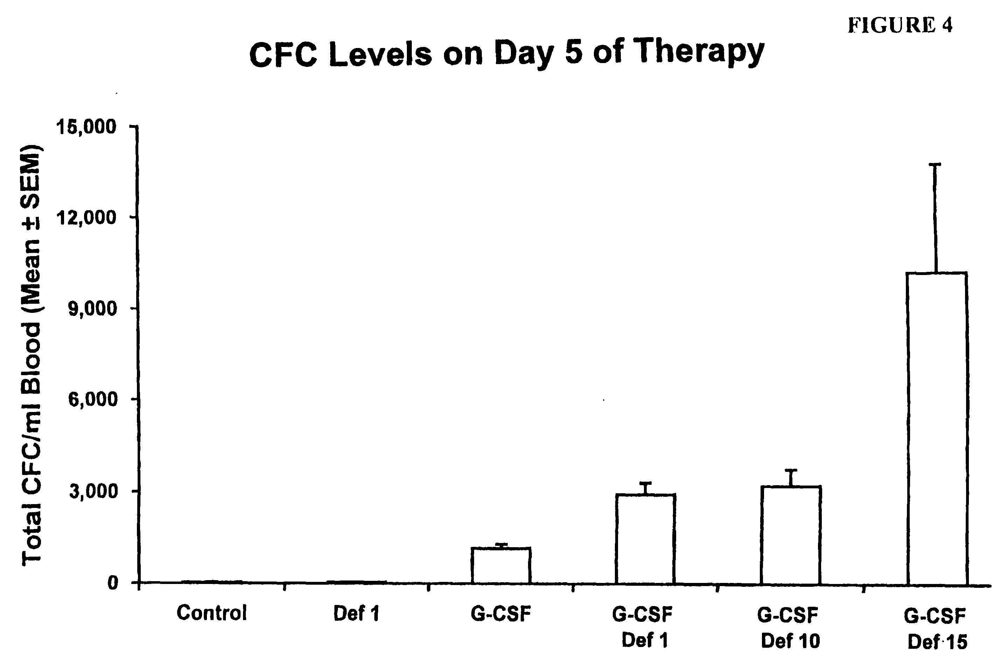

Total CFCs (CFU-GM + BFU-E + CFU-Mix + HPP-CFC) per millilitre of blood after 5 days' treatment of the mice of Example 1; the data are given in Figure 4 and are expressed as mean ± SEM derived from duplicated cultures on samples from each animal.

### EXAMPLE 5

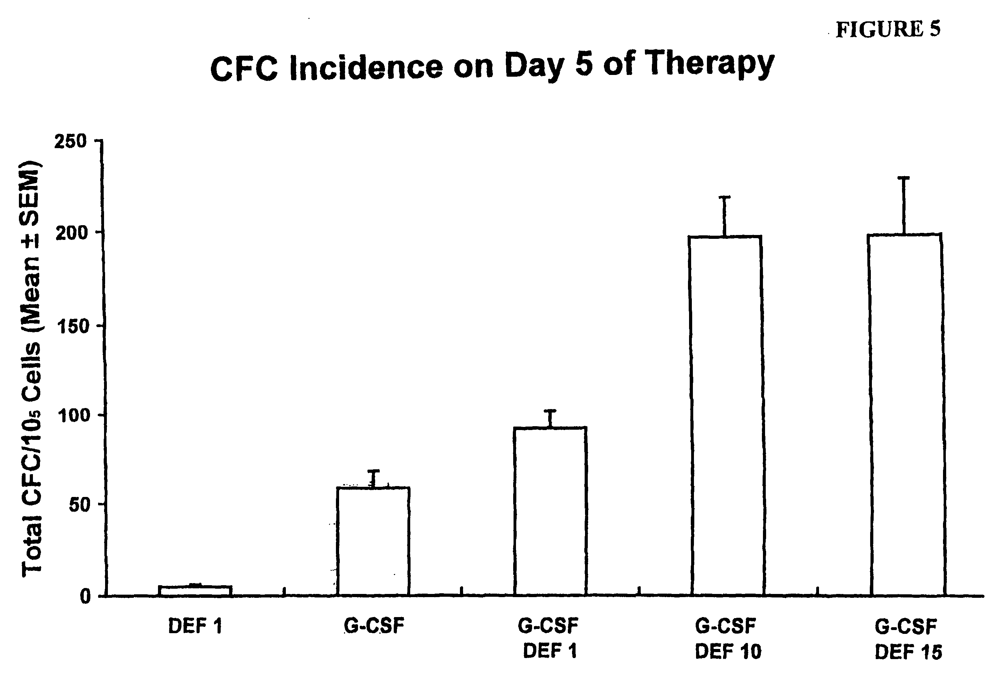

Frequency of the total CFCs (CFU-GM + BFU-E + CFU-Mix + HPP-CFC) for 10⁵ buffy-coat cells of peripheral blood in the mice mentioned in Example 1; the data are given in Figure 5 and are expressed as mean ± SEM derived from duplicated cultures on samples from each animal.

### EXAMPLE 6

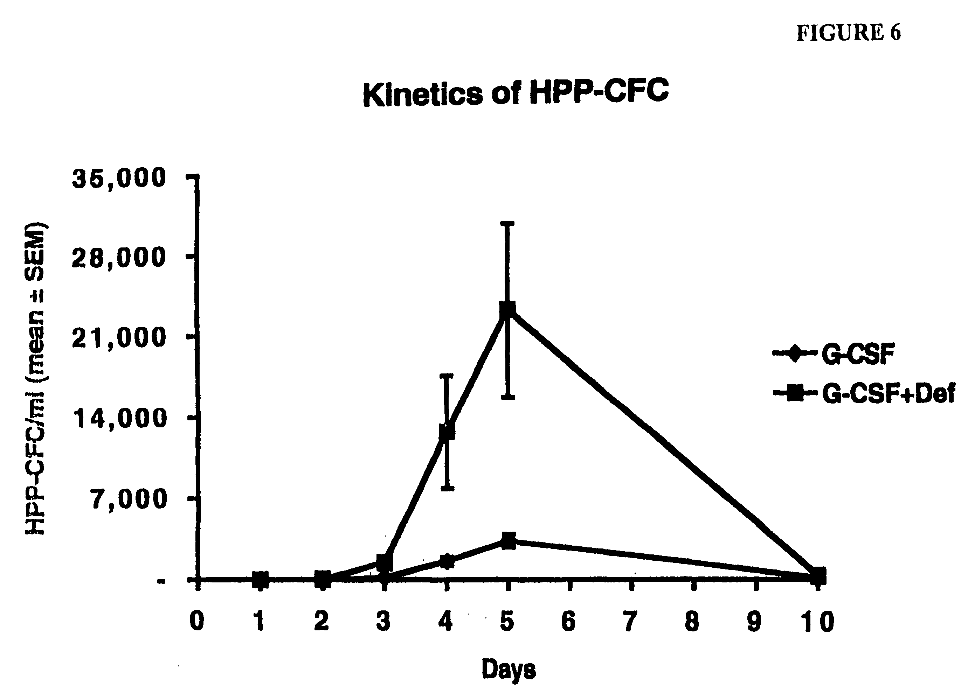

This experiment was carried out to evaluate the effect of the administration of G-CSF and/or DEF on the amount of haematopoietic progenitors/stem cells (High-Proliferative Potentia-Colony Stimulating Cells, or HPP-CFC) in circulation in the peripheral blood of monkeys. The study was carried out in rhesus monkeys (Macaca Mulatta) of 4-6 years in good health and which showed normal values for hematology and clinical chemistry. The G-CSF was dosed subcutaneously, 100 µg/kg, for 5 days for two cycles; DEF was dosed 15 mg/kg/hr by a continuous infusion system for 5 days at the second cycle. The animals were anaesthetised for bleeding, administration of G-CSF and changing the drug-bag. The results of this experiment are given in Figure 6, from which it can be appreciated that the combined administration of G-CSF and defibrotide enables an HPP-CFC mobilisation in monkeys which is about 8 times higher than that obtainable by the administration of G-CSF alone.

### CONCLUSIONS

As will be readily appreciated from the data given in Figure 1, the combined administration of G-CSF and defibrotide produces a substantial increase in the amount of white blood cells in circulation in murine blood. It should, in particular, be noted that the administration of defibrotide alone does not have a positive influence on the amount of white blood cells in circulation; the combination of G-CSF and defibrotide therefore produces a surprising dose-dependent effect of increasing the number of white blood cells, which is not merely the sum of two effects which are independent of one another.

Figures 2 to 5 clearly indicate that the combined administration of G-CSF and defibrotide enables levels of mobilisation to be obtained in mice that are from 10 to 100 times higher than those obtainable by the administration of G-CSF alone.

Finally, figure 6 confirms that the combined administration of G-CSF and defibrotide enables a stem cell (HPP-CFC) mobilisation in monkeys which is about 8 times higher than that obtainable by the administration of G-CSF alone.

The combined effect of the two active ingredients is dose-dependent because the levels of mobilisation increase proportionally with the amount of defibrotide administered; the highest mobilisation peak is reached in all cases on approximately the fifth day from administration.

## Claims

1. A formulation containing as active agents defibrotide and at least one haematopoietic factor having the capacity to mobilise haematopoietic progenitors.

2. A formulation according to claim 1, **characterised in that** the haematopoietic factor having the capacity to mobilise haematopoietic progenitors is G-CSF.

3. A formulation according to claim 1, **characterised in that** it is an aqueous solution.

4. A formulation according to claim 1, constituted by two different separately administrable formulations, one containing the haematopoietic factor having the capacity to mobilise haematopoietic progenitors and the other containing defibrotide.

5. A formulation containing defibrotide and at least one haematopoietic factor having the capacity to mobilise haematopoietic progenitors, preferably G-CSF, as a combined preparation for simultaneous, separate or sequential use to increase the amount of stem cells and/or haematopoietic progenitors in the peripheral blood of a mammal.

6. A formulation according to claims 1-5 further containing the usual excipients and/or coadjuvants.

7. The use of defibrotide in combination with at least one haematopoietic factor having the capacity to mobilise haematopoietic progenitors for the preparation of formulations capable of increasing the amount of stem cells and/or haematopoietic progenitors in the peripheral blood of a mammal.

8. The use according to claim 7, **characterised in that** the haematopoietic factor having the capacity to mobilise haematopoietic progenitors is G-CSF.

## Patentansprüche

1. Formulierung, enthaltend als Wirkstoffe Defibrotid und mindestens einen hämatopoetischen Faktor mit der Fähigkeit, hämatopoetische Vorläuferzellen zu mobilisieren.

2. Formulierung gemäß Anspruch 1, **dadurch gekennzeichnet, dass** der hämatopoetische Faktor mit der Kapazität hämatopoetische Vorläuferzellen zu mobilisieren, G-CSF ist.

3. Formulierung gemäß Anspruch 1, **dadurch gekennzeichnet, dass** sie eine wässrige Lösung ist.

4. Formulierung gemäß Anspruch 1, aufgebaut aus zwei unterschiedlichen, getrennt verabreichbaren Formulierungen, wobei eine den hämatopoetischen Faktor mit der Kapazität, hämatopoetische Vorläuferzellen zu mobilisieren, enthält, und die andere Defibrotid enthält.

5. Formulierung, enthaltend Defibrotid und mindestens einen hämatopoetischen Faktor mit der Fähigkeit, hämatopoetische Vorläuferzellen zu mobilisieren, vorzugsweise G-CSF, als eine kombinierte Präparation zur gleichzeitigen, getrennten oder aufeinanderfolgenden Verwendung zur Erhöhung der Menge von Stammzellen und/oder hämatopoetischen Vorläuferzellen im peripheren Blut eines Säugers.

6. Formulierung gemäß den Ansprüchen 1 bis 5, ferner die üblichen Arzneistoffträger und/oder Co-Hilfsstoffe enthaltend.

7. Verwendung von Defibrotid in Kombination mit mindestens einem hämatopoetischen Faktor mit der Fähigkeit, hämatopoetische Vorläuferzellen zu mobilisieren, zur Herstellung von Formulierungen, die zur Erhöhung der Menge von Stammzellen und/oder hämatopoetischen Vorläuferzellen im peripheren Blut eines Säugers in der Lage sind.

8. Verwendung gemäß Anspruch 7, damit **gekennzeichnet**, dass der hämatopoetische Faktor mit der Fähigkeit, hämatopoetische Vorläuferzellen zu mobilisieren, G-CSF ist.

## Revendications

1. Formulation contenant comme ingrédients actifs du défibrotide et au moins un facteur hématopoïétique ayant la capacité de mobiliser les progéniteurs hématopoïétiques.

2. Formulation selon la revendication 1, **caractérisée en ce que** le facteur hématopoïétique ayant la capacité de mobiliser les progéniteurs hématopoïétiques est G-CSF.

3. Formulation selon la revendication 1, **caractérisée en ce qu'**elle est une solution aqueuse.

4. Formulation selon la revendication 1, constituée par deux formulations administrables séparément différentes, l'une contenant le facteur hématopoïétique ayant la capacité de mobiliser les progéniteurs hématopoïétiques et l'autre contenant le défibrotide.

5. Formulation contenant du défibrotide et au moins un facteur hématopoïétique ayant la capacité de mobiliser les progéniteurs hématopoïétiques, de préférence G-CSF, sous forme d'une préparation combinée pour usage simultané, séparé ou séquentiel pour augmenter la quantité de cellules souches et/ou de progéniteurs hématopoïétiques dans le sang périphérique d'un mammifère.

6. Formulation selon les revendications 1 à 5 contenant en outre les excipients et/ou coadjuvants usuels.

7. Utilisation du défibrotide en combinaison avec au moins un facteur hématopoïétique ayant la capacité de mobiliser les progéniteurs hématopoïétiques pour la préparation de formulation pouvant augmenter la quantité de cellules souches et/ou de progéniteurs hématopoïétiques dans le sang périphérique d'un mammifère.

8. Utilisation selon la revendication 7, **caractérisée en ce que** le facteur hématopoïétique ayant la capacité de mobiliser les progéniteurs hématopoïétiques est G-CSF.
